# EUROPÄISCHE PATENTSCHRIFT

(11) **EP 1 709 955 B1**
(45) Veröffentlichungstag und Bekanntmachung des Hinweises auf die Patenterteilung: **18.07.2007**
(21) Anmeldenummer: 05007721.3
(22) Anmeldetag: 08.04.2005
(51) Int. Cl.: A61K 8/368

(54) **Konzentrierte, wässrige Lösungen von p-Methoxybenzoesäure für den Einsatz in kosmetischen und dermatologischen Formulierungen**
Concentrated, aqueous solutions of p-methoxybenzoic acid for use in cosmetic and dermatological formulations
Solutions aqueuses concentrées d' acide p-methoxybenzoique destinés à être utilisés dans de produits cosmétiques ou dermatologiques

(43) Veröffentlichungstag der Anmeldung: 11.10.2006
(73) Patentinhaber: Dr. Straetmans GmbH, 22143 Hamburg (DE)
(72) Erfinder: Straetmans, Udo, 22149 Hamburg (DE); Jänichen, Jan, 22143 Hamburg (DE); Petersen, Wilfried, 22143 Hamburg (DE); Kinder, Michael, 20253 Hamburg (DE); Johnson, Christopher H., Hazlet, NJ 07730 (US); Reynolds, Garrett S., Hazlet, NJ 07730 (US)
(74) Vertreter: von Eichel-Streiber, Caspar

(56) Entgegenhaltungen:
- WO-A-87/06827
- FR-A- 2 834 459
- N. GULYAEVA ET AL: "Measurement of the Relative Hydrophobicity of Organic Compounds Without Organic Solvent. Effects of Salt Composition and pH on Organic Acids and Nonionic Compounds" JOURNAL OF PHARMACEUTICAL SCIENCES, Bd. 90, Nr. 9, September 2001 (2001-09), Seiten 1366-1374, XP002353574

## Beschreibung

### Technischer Hintergrund der Erfindung

Die Optimierung von Produktionsprozessen zur Einsparung von Zeit, Raum oder Ressourcen wie Energie oder Arbeitskraft definiert das Aufgabengebiet des technischen Betriebscontrollings und gehört mit zu den wirksamsten Möglichkeiten einer Verbesserung der Kostensituation im produzierenden Gewerbe. Unter dem Druck einer Reduktion von Stückkosten wird dabei kontinuierlich versucht, Arbeitsprozesse auf allen Ebenen effizienter zu gestalten. Neben der Verbesserung interner Abläufe werden dabei auch externe Partner wie Rohstoff-lieferanten in immer stärkerem Maße in die Optimierung von Produktionsprozessen eingebunden. So kann z.B. eine zeitgenaue Bereitstellung von Rohstoffen in produktionskompatiblen Rohstoffgebinden Lagerkapazitäten auf die Straße verlagern, den Arbeitsfluss nachhaltig verbessern und zu einer signifi-anten Reduktion der Stückkosten beitragen.

Ein wichtiger Aspekt, der insbesondere in größeren Produktionsinheiten eine signifikante Rolle spielen kann, ist der Aggregatzustand eines Rohstoffs. Während flüssige oder gasförmige Materialien durch Pumpen leicht und gut dosierbar dem Produktionsprozess zugeführt werden können, ist das Einbringen fester Rohstoffe gewöhnlich mit Wägeprozessen und einer offenen Produkthandhabung verbunden. Durch letzteren Punkt werden auch Sicherheitsaspekte berührt, da das in der Produktion tätige Personal dem offenen Produkt ausgesetzt wird.

Feste und schwerlösliche Rohstoffe, die vor der Weiterverarbeitung in eine gelöste Form gebracht werden müssen, stellen zusätzliche Anforderungen an die Produktionsprozesse, denn diese Lösungsvorgänge erfordern oftmals die Zuführung von Energie, benötigen viel Zeit und in vielen Fällen zusätzliche Mischbehälter. Von daher werden viele schwerlösliche Rohstoffe zwar in Labor und Entwicklung gerne eingesetzt, scheitern aber an ihrer schwierigen Verarbeitbarkeit in Produktionsprozessen.

Vor diesem Hintergrund sind flüssige und konzentrierte Formulierungen schwerlöslicher Rohstoffe, die nicht mit den Qualitätsanforderungen des Endproduktes in Konflikt stehen, von offensichtlichem technischem und ökonomischem Vorteil und somit Ziel der Erfindung.

### Gegenstand der vorliegenden Erfindung

p-Methoxybenzoesäure hat in den vergangenen Jahren als multifunktioneller Rohstoff in der kosmetischen und in der Lebensmittelindustrie eine wachsende Bedeutung erlangt. So sind z.B. wässrige Zusammensetzungen und deren Verwendung in Kosmetika bekannt (FR-A-2 834 459), die 1-30% p-Methoxybenzoesäure und bis zu 50% eines oder mehrerer Polyole, wie Glycerin, enthalten (siehe Ansprüche 1-24). Eine Base wie Natriumhydroxid, und Zitronensäure ist auch darin enthalten. Die Zusammensetzungen haben ein pH von 5.2. Neben seiner Hauptfunktion als Maskierungsagens und Aromakomponente haben die interessanten Eigenschaften dieses Rohstoffes zur biologischen Stabilisierung von kosmetischen und dermatologischen Formulierungen und als Wirkstoff gegen spezifische Keime in Produkten der Haut- und Haarpflege ein großes Interesse an diesem Rohstoff geweckt. In wässrigen Systemen ist die Löslichkeit der p-Methoxybenzoesäure sehr gering und der Lösungsvorgang selbst ausgesprochen langsam, was die Einarbeitung der p-Methoxybenzoesäure in kosmetischen Formulierungen erschwert. Als Umweg bietet sich für gewöhnlich eine Einarbeitung über die Form der löslicheren Salze an. Die Kalium- und Natriumsalze der p-Methoxybenzoesäure gehen in wässriger Umgebung dabei zunächst leicht in Lösung und können in die Formulierung eingearbeitet werden. Die Salze können durch späteres Ansäuern wieder in die aktive p-Methoxybenzoesäure zurück überführt werden.
Jedoch existieren offenbar von diesen Salzen Kristallformen von geringerer Löslichkeit, die insbesondere bei höheren Konzentrationen und in der Kälte nach einiger Zeit irreversibel auskristallisieren. Die Herstellung und Lagerung höher konzentrierter, wässriger Lösungen ist daher bislang nicht möglich gewesen, würde jedoch einen erheblichen technischen Fortschritt bedeuten.
Überraschenderweise wurde nun gefunden, dass den Nachteilen des Standes der Technik Abhilfe geschaffen werden kann, indem eine flüssige, mit einer Base in etwa neutralisierte Kombination nach Anspruch 1 vorgesehen wird. In diesen Lösungen kann es zwar bei niedrigen Temperaturen zur Kristallbildung kommen, diese ist jedoch reversibel und beim Erwärmen auf Raumtemperatur wird im Gegensatz zu rein wässrigen Systemen erneut eine klare und homogene Lösung gebildet.

Zur Gruppe der Polyole, die eine erfindungsgemäße Stabilisierung bewirken können, gehören Glycerin oder Glykole wie z.B. Propylenglykol, Butylenglykol, Dipropylenglykol oder 2-Methylpropandiol. Geeignet sind ferner Polyglycerinester, wie z.B. Polyglyceryl-10 Laurate oder Sorbitanester, wie z.B. Sorbitan Laurate. Besonders geeignet ist dabei Glycerin.

Salze geeigneter organische Säuren, die sich für eine erfindungsgemäße Stabilisierung eignen sind dabei die Natrium- und Kaliumsalze der Lävulinsäure, Milchsäure oder Zitronensäure sowie Ammoniumsalze von allgemeinen Typ HNR₃⁺ mit R = H, alkyl, hydroxyalkyl. Besonders bevorzugt sind die Kalium- und Natriumsalze der Lävulinsäure.

### Beschreibung der Erfindung

p-Methoxybenzoesäure kann unter dem Handelsnamen Dermosoft^{®} 688 von der Dr. Straetmans GmbH in kosmetikkonformer Qualität bezogen werden.
Die Löslichkeit von p-Methoxybenzoesäure in Wasser liegt bei etwa 0,5 g/l. Zur Einarbeitung in eine kosmetische Formulierung wird das Produkt gewöhnlich mit Natronlauge oder Kalilauge in wenig Wasser in der Wärme in Lösung gebracht und in die wässrige Phase eingearbeitet. Um das Wirkspektrum der p-Methoxybenzoesäure voll zur Entfaltung zu bringen, sollte die Formulierung anschließend wieder auf einen pH-Wert von < 5,5 angesäuert werden.
In der Literatur werden die Salze der p-Methoxybenzoesäure als gut löslich in Wasser beschrieben, jedoch zeigt sich in der Praxis, dass es bei einer Einlagerung höher konzentrierter Lösungen der Kalium- oder Natriumsalze für längere Zeit und /oder bei niedriger Temperatur zu einer Kristallisation der Salze kommen kann. Diese gehen überraschenderweise beim nachfolgenden Erwärmen auf Raumtemperatur nicht wieder in Lösung, was daraufhin deutet, dass es sich bei den zunächst gebildeten homogenen Lösungen nur um metastabile Systeme handelt.

Dem Stand der Technik entsprechend kann die Löslichkeit von Salzen durch den Zusatz fremdionischer Salze verbessert werden. Der Theorie folgend, werden durch die Zugabe solcher Salze die chemische Aktivität und damit das Lösungsvermögen des Lösungsmittels verbessert. Die Zugabe gleichionischer Zusätze hingegen, sowie die Zugabe von Lösungsmitteln, die nicht oder nur in geringem Maße zur Solvatisierung von Ionen befähigt sind, sollte der Theorie entsprechend die Löslichkeit der zu lösenden Salze herabsetzen.

Völlig überraschend und der Theorie folgend nicht zu erwarten, wurde nun gefunden, dass die Löslichkeit der Natrium- und Kaliumsalze der p-Methoxybenzoesäure in Gegenwart von organischen Lösungsmitteln aus der Gruppe der Polyole verbessert werden kann. Als besonders geeignet hat sich dabei Glycerin erwiesen, aber auch Glykole wie Propylenglykol, Butylenglykol, Dipropylenglykol oder 2-Methylpropandiol konnten die Löslichkeit verbessern. Geeignet sind ferner Solubilisatoren aus der Gruppe der Polyglycerinester, wie z.B. Polyglyceryl-10 Laurate oder aus der Gruppe der Sorbitanester, wie z.B. Sorbitan Laurate.

So erwiesen sich beispielsweise folgende Lösungen als thermodynamisch stabil:

### Lösung 1

| | |
|---|---|
| 5 g | p-Methoxybenzoesäure |
| 30 g | Glycerin |
| 15,0 g | 10 % Natriumhydroxid in Wasser |
| ad 100 g | Wasser |
| pH = 10 - 12 | |

### Lösung 2

| | |
|---|---|
| 5 g | p-Methoxybenzoesäure |
| 31,5 g | Polyglyceryl-10 Laurate |
| 14,0 g | 10 % Natriumhydroxid in Wasser |
| ad 100 g | Wasser |
| pH = 7,5 | |

Ebenso verbessert die Gegenwart von Salzen organischer Säuren die Löslichkeit der genannten Salze der p-Methoxybenzoesäure. Hier haben sich insbesondere Natrium- und Kaliumlävulinat sowie die Ammoniumsalze der Lävulinsäure von allgemeinen Typ HNR₃⁺ mit R = H, alkyl, hydroxyalkyl als geeignet herausgestellt, aber auch die entsprechenden Lactate und Citrate erwiesen sich als geeignet.
Am effektivsten wird die Löslichkeit der Salze der p-Methoxybenzoesäure verbessert und dadurch dem Stand der Technik Abhilfe geschaffen durch eine Kombination von Polyolen und den genannte Salzen der Lävulinsäure. Beispielhaft sein hier die folgenden Lösungen erwähnt

### Lösung 3

| | |
|---|---|
| 5 g | p-Methoxybenzoesäure |
| 21 g | Lävulinsäure |
| ~ 19 g | 45 %ige Natriumhydroxid Lösung in Wasser |
| 50 g | Glycerin |
| ad 100 g | Wasser |
| pH = 7,5 | |

### Lösung 4

| | |
|---|---|
| 7,5 g | p-Methoxybenzoesäure |
| 22,5 g | Lävulinsäure |
| ~ 21,5 g | 45 %ige Natriumhydroxid Lösung in Wasser |
| 25 g | Glycerin |
| ad 100 g | Wasser |
| pH = 7,5 | |

Die genannten erfindungsgemäßen Gemische sind über lange Zeit lagerstabil und zeigen keinerlei Tendenz zu irreversibler Kristallisation. Diese Gemische zeigen überdies die vorteilhafte Eigenschaft, kosmetische Formulierungen gegenüber einem Wachstum von Bakterien, Hefen und Pilzen gleichermaßen ausgewogen zu stabilisieren.

Die Gemische können vorteilhaft hergestellt werden, indem die p-Methoxybenzoesäure zunächst in dem Polyol dispergiert wird, anschließend mit einer auf den Gesamtsäuregehalt berechneten, stöchiometrischen Menge einer wässrigen Lösung der Base versetzt wird. In Gemischen die weitere organische Säuren enthalten, dienen diese zu Einstellung des pH-Wertes der Mischung auf einen Wert zwischen 7,0 und 8,0.

Gegenstand der Erfindungen sind demzufolge wässrige Lösungen der Natrium-, Kalium- oder Ammoniumsalze von allgemeinen Typ HNR₃⁺ mit R = H, alkyl, hydroxyalkyl der p-Methoxybenzoesäure, die durch Zusatz von Polyolen und / oder den Natrium-, Kalium- oder Ammoniumsalze von allgemeinen Typ HNR₃⁺ mit R = H, alkyl, hydroxyalkyl der Lävulinsäure, Milchsäure oder Zitronensäure stabilisiert wurden, deren Herstellung sowie deren Einsatz zur Beduftung, Maskierung, Aromatisierung und biologischen Stabilisierung von kosmetischen oder dermatologischen Formulierungen zur Behandlung, Pflege oder Reinigung der Haut und / oder der Haare sowie in Produkten der dekorativen Kosmetik.

## Patentansprüche

1. Flüssige, mit einer Base in etwa neutralisierte Kombination, enthaltend:
a. 5 - 15 Gew.% p- Methoxybenzoesäure
b. 5 - 60 Gew.% Wasser
c. 20 - 30 Gew.% organische Säure(n) oder deren Salze und/oder
d. 20 - 60 Gew.% Polyole und/oder Polyglycerinester und/oder Sorbitanester,
ergänzend zu oder anstelle von c.

2. Kombinationen gemäß Anspruch 1, bestehend aus
a. 5 Gew.% bis 10 Gew.% Natriumsalz der p-Methoxybenzoesäure
b. 20 Gew.% bis 30 Gew.% Natriumlävulinat
c. 20 Gew.% bis 55 Gew.% Glycerin
d. 5 Gew.% bis 55 Gew.% Wasser

3. Herstellung der Kombination gemäß einem der Ansprüche 1 und 2 durch Dispergieren der p-Methoxybenzoesäure in einem Polyol, vorzugsweise Glycerin, nachfolgender Zugabe der auf die Gesamtsäuremenge bezogenen Äquivalente einer Base, vorzugsweise von Natriumhydroxid, Einstellung eines pH-Wertes von 7,0 - 8,0 mit einer organischen Säure, vorzugsweise Lävulinsäure, und ggf. Zugabe des restlichen Wassers.

4. Verwendung einer Kombinationen gemäß einem Ansprüche 1 und 2 in kosmetischen oder dermatologischen Formulierungen zur Behandlung, Pflege oder Reinigung der Haut und/oder der Haare sowie in Produkten der dekorativen Kosmetik mit dem Zweck der Beduftung, Maskierung, Aromatisierung oder biologischen Stabilisierung.

## Claims

1. Liquid, in neutralized combination with a base, containing:
a. 5 to 15 wt.% p-methoxybenzoic acid,
b. 5 to 60 wt.% water,
c. 20 to 30 wt.% organic acid(s) or their salts and/or
d. 20 to 60 wt.% polyols and/or polyglycerol esters and/or sorbitan esters, in addition to or in place of c.

2. Combinations according to claim 1, comprising
a. 5 to 10 wt.% p-methoxybenzoic acid sodium salt,
b. 20 to 30 wt.% sodium levulinate,
c. 20 to 55 wt.% glycerol,
d. 5 to 55 wt.% water.

3. Preparation of the combination according to one of the claims 1 and 2 by dispersing the p-methoxybenzoic acid in a polyol, preferably glycerol, subsequent addition of the equivalents of a base based on the total acid quantity and preferably sodium hydroxide, setting a pH-value of 7 to 8 with an organic acid, preferably levulinic acid and optionally adding the remaining water.

4. Use of one of the combinations according to one of the claims 1 and 2 in cosmetic or dermatological formulations for the treatment, care or cleaning of the skin and/or hair and in decorative cosmetic products for perfuming, masking, aromatizing or biological stabilizing purposes.

## Revendications

1. Combinaison liquide, à peu près neutralisée avec une base, contenant :
a. 5-15 % en poids d'acide p-méthoxybenzoïque
b. 5-60 % en poids d'eau
c. 20-30 % en poids d'un ou plusieurs acides organiques ou leurs sels et/ou
d. 20-60 % en poids de polyols et/ou de polymères d'esters de glycérol et/ou d'un ester de sorbitanne,
en complément ou à la place de c.

2. Combinaisons suivant la revendication 1, constituée de
a. 5 % en poids à 10 % en poids de sel de sodium d'acide p-méthoxybenzoïque
b. 20 % en poids à 30 % en poids de lévulinate de sodium
c. 20 % en poids à 55 % en poids de glycérol
d. 5 % en poids à 55 % en poids d'eau.

3. Préparation de la combinaison suivant l'une des revendications 1 et 2 par dispersion de l'acide p-méthoxybenzoïque dans un polyol, avantageusement le glycérol, suivie de l'addition de l'équivalent, rapporté à la quantité totale d'acide, d'une base, avantageusement l'hydroxyde de sodium, ajustement du pH à une valeur de 7,0 à 8,0 avec un acide organique, avantageusement l'acide lévulinique et, le cas échéant, addition de la quantité restante d'eau.

4. Utilisation d'une combinaison selon l'une des revendications 1 et 2 dans des formulations cosmétiques ou dermatologiques en vue du traitement, des soins ou du nettoyage de la peau et/ou des cheveux, ainsi que dans des produits de maquillage à des fins d'apport de parfum, de masquage, d'aromatisation ou de stabilisation biologique.
